# EUROPEAN PATENT APPLICATION

(11) **EP 2 243 843 A1**
(43) Date of publication of application: **27.10.2010**
(21) Application number: 09305351.0
(22) Date of filing: 24.04.2009
(51) Int. Cl.: C12Q 1/68

(54) **HP1alpha as a prognostic marker in human cancer**

(71) Applicant: Institut Curie, 75248 Paris Cedex 05 (FR); Centre National de la Recherche Scientifique, 75016 Paris (FR)
(72) Inventor: Almouzni, Geneviève, 92200 Neuilly sur Seine (FR); De Koning, Leanne, 92290 Chatenay Malabry (FR)
(74) Representative: Gallois, Valérie

(57) **Abstract**

The present invention provides a prognostic marker in human cancer, HP1α, a high expression thereof being associated with a poor prognosis. The present invention further provides a method for diagnosing a cancer in a subject, HP1α being specifically overexpressed in tumoral cells. The present invention also provides a method for selecting a subject affected with a cancer for an adjuvant therapy and a method for monitoring the response of a subject affected with a cancer to a treatment.

SEQUENCE LISTING

<110>  Institut Curie
       CNRS

<120>  HP1alpha as a prognostic marker in human cancer

<130>  B866EP

<160>  13

<170>  PatentIn version 3.3

<210>  1
<211>  21
<212>  RNA
<213>  Artificial

<220>
<223>  siRNA GFP

<400>  1
aagcuggagu acaacuacaa c                                                21


<210>  2
<211>  21
<212>  DNA
<213>  Artificial

<220>
<223>  siRNA HP1alpha_1

<400>  2
ccugagaaaa acuuggauut t                                                21


<210>  3
<211>  19
<212>  DNA
<213>  Artificial

<220>
<223>  siRNA HP1alpha_3

<400>  3
gggagaagtc agaaagtaa                                                   19


<210>  4
<211>  19
<212>  DNA
<213>  Artificial

<220>
<223>  siRNA HP1beta

<400>  4
aggaatatgt ggtggaaaa                                                   19


<210>  5
<211>  19
<212>  DNA
<213>  Artificial

<220>
<223>  siRNA HP1gamma

<400>  5

aggtcttgat cctgaaaga                                                    19

<210>  6
<211>  20
<212>  DNA
<213>  Artificial

<220>
<223>  RPLPO forward primer

<400>  6
ggcgacctgg aagtccaact                                                   20

<210>  7
<211>  20
<212>  DNA
<213>  Artificial

<220>
<223>  RPLPO reverse primer

<400>  7
ccatcagcac cacagccttc                                                   20

<210>  8
<211>  20
<212>  DNA
<213>  Artificial

<220>
<223>  HP1alpha forward primer

<400>  8
gatcattggg gcaacagatt                                                   20

<210>  9
<211>  20
<212>  DNA
<213>  Artificial

<220>
<223>  HP1alpha reverse primer

<400>  9
tgcaagaacc aggtcagctt                                                   20

<210>  10
<211>  20
<212>  DNA
<213>  Artificial

<220>
<223>  CAF-1 p150 forward primer

<400>  10
cagcagtacc agtcccttcc                                                   20

<210>  11
<211>  22
<212>  DNA
<213>  Artificial

<220>
<223>  CAF-1 p150 reverse primer

<400>  11
tctttgcagt ctgagcttgt tc                                                22

<210>  12
<211>  20
<212>  DNA
<213>  Artificial

<220>
<223>  CAF-1 p60 forward primer

<400>  12
cggacactcc accaagttct                                                   20

<210>  13
<211>  20
<212>  DNA
<213>  Artificial

<220>
<223>  CAF-1 p60 reverse primer

<400>  13
ccaggcgtct ctgactgaat                                                   20

## Description

### FIELD OF THE INVENTION

The present invention relates to the field of medicine, in particular of oncology. It provides a new prognostic and/or diagnostic marker in human cancer.

### BACKGROUND OF THE INVENTION

Cancer occurs when cell division gets out of control and results from impairment of a DNA repair pathway, the transformation of a normal gene into an oncogene or the malfunction of a tumor supressor gene. Many different forms of cancer exist. The incidence of these cancers varies but it represents the second highest cause of mortality, after heart disease, in most developed countries. While different forms of cancer have different properties, one factor which many cancers share is the ability to metastasize. Distant metastasis of all malignant tumors remains the primary cause of death in patients with the disease.

The therapeutic care of the patients having cancer is primarily based on surgery, radiotherapy and chemotherapy and the practitioner has to choose the most adapted therapeutic strategy for the patient. In the majority of the cases, the choice of the therapeutic protocol is based on the anatomo-pathological and clinical data. Currently, the methods to determine prognosis and select patients for adjuvant therapy rely mainly on pathological and clinical staging. However, it is very difficult to predict which localized tumor will eventuate in distant metastasis.

Therefore, there is a great need for the identification of prognostic markers that can accurately distinguish tumors associated with poor prognosis including high probability of metastasis, early disease progression, increased disease recurrence or decreased patient survival, from the others. Using such markers, the practitioner can predict the patient's prognosis and can effectively target the individuals who would most likely benefit from adjuvant therapy.

### SUMMARY OF THE INVENTION

The inventors demonstrate that HPlα is overexpressed in cancer cells in comparison with non-tumoral cells and that high HP1α expression levels in cancer cells are correlated with a poor prognosis including early disease progression, increased metastasis formation, increased disease recurrence and/or decreased patient survival.

Accordingly, in a first aspect, the present invention concerns a method for predicting or monitoring clinical outcome of a subject affected with a cancer, wherein the method comprises the step of determining the expression level of HP1α in a cancer sample from said subject, a high expression level of HP1α being indicative of a poor prognosis. Preferably, a poor prognosis is a decreased patient survival and/or an early disease progression and/or an increased disease recurrence and/or an increase metastasis formation.

In a second aspect, the present invention concerns a method for diagnosing a cancer in a subject, wherein the method comprises the step of determining the expression level of HP1α in a sample from said subject, a high expression level of HP1α indicating that said subject suffers from a cancer.

In a third aspect, the present invention concerns a method for selecting a subject affected with a cancer for an adjuvant chemotherapy and/or radiotherapy or determining whether a subject affected with a cancer is susceptible to benefit from an adjuvant chemotherapy and/or radiotherapy, wherein the method comprises the step of determining the expression level of HP1α in a cancer sample from said subject, a high expression level of HP1α indicating that an adjuvant chemotherapy and/or radiotherapy is required.

In a last aspect, the present invention concerns a method for monitoring the response to a treatment of a subject affected with a cancer, wherein the method comprises the step of determining the expression level of HP1α in a cancer sample from said subject before the administration of the treatment , and in a cancer sample from said subject after the administration of the treatment, a decreased expression level of HP1α in the sample obtained after the administration of the treatment indicating that the subject is responsive to the treatment.

In one embodiment, the expression level of HP1α is determined by measuring the quantity of HP1α protein or HP1α mRNA.

In a further embodiment, the quantity of HP1α protein is measured by immunohistochemistry or semi-quantitative Western-blot.

In another further embodiment, the quantity of HP1α mRNA is measured by quantitative or semi-quantitative RT-PCR, or by real time quantitative or semi-quantitative RT-PCR

In another embodiment, the methods of the invention comprise the step of comparing the expression level of HP1α to a reference expression level. Additionally, the methods of the invention further comprise the step of determining whether the expression level of HP1α is high compared to said reference expression level.

In a further embodiment, the reference expression level is the expression level of HP1α in a normal sample.

Alternatively, the reference expression level is the expression level of a gene having a stable expression in different cancer samples, such as RPLPO gene.

In still another embodiment, the method according to the invention further comprises assessing at least one another cancer or prognosis markers such as tumor grade, hormone receptor status, mitotic index, tumor size or expression of proliferation markers such as Ki67, MCM2, CAF-1 p60 and CAF-1 p150.

In still another embodiment, the cancer is a solid cancer or a hematopoietic cancer, preferably a solid cancer and more preferably, an early stage solid cancer without local or systemic invasion.

Preferably, the cancer is selected from the group consisting of breast cancer, pancreas cancer, uterus and cervix cancers, ovary cancer, prostate cancer and leukemia. More preferably, the cancer is breast cancer. Even more preferably, the cancer is an early stage breast cancer without local or systemic invasion.

### BRIEF DESCRIPTION OF THE DRAWINGS

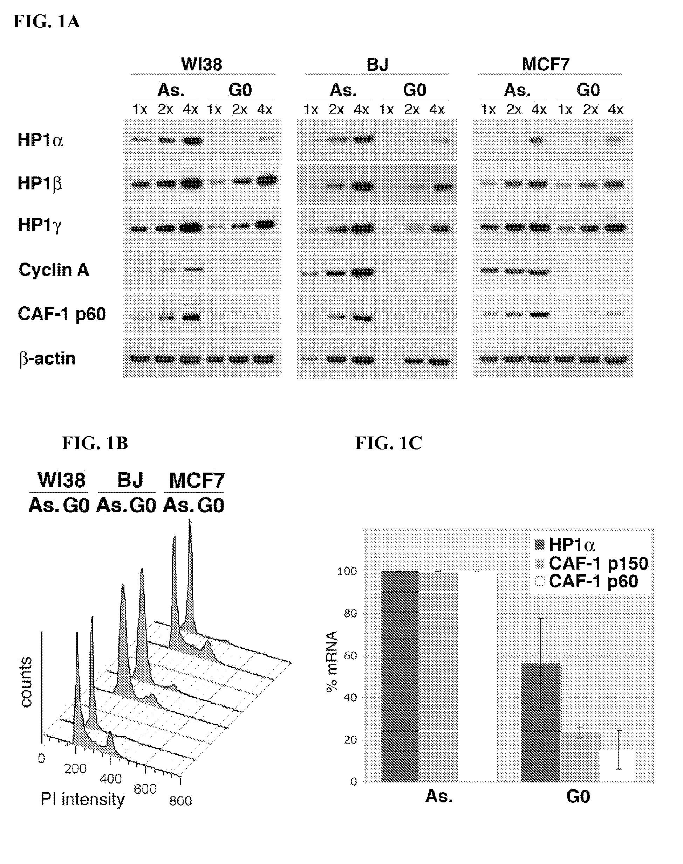

**Figure 1****: The expression of HP1**α**, but not** β **or** γ**, is downregulated in quiescence.** ***Fig 1A******.*** Total protein levels of HP1α, β and γ are detected by Western Blot in asynchronously proliferating (As.) and quiescent (G0) WI38 lung fibroblasts, MCF7 breast cancer cells and BJ primary foreskin fibroblasts. Fibroblasts are arrested in quiescence by serum starvation and MCF7 cells by anti-estrogen treatment. Increasing amounts (x) of total cells extracts are loaded and β-action serves as a loading control. CAF-1 p60 (Polo et al, 2004) and Cyclin A are used as markers for cell proliferation. ***Fig 1B****.* Flow cytometry analysis of the cell cycle distribution of the cells shown in *A.* ***Fig 1*****C***.* HP1α mRNA levels in proliferating (As.) and quiescent (G0) BJ foreskin fibroblasts, as determined by quantitative RT-PCR. Levels are normalized to the reference gene ribosomal protein P0-like protein (RPLPO) (de Cremoux et al, 2004) and levels in proliferating cells are set to 100%. CAF-1 p60 and CAF-1 p150 levels are shown for comparison. Error bar represents data from three independent experiments.

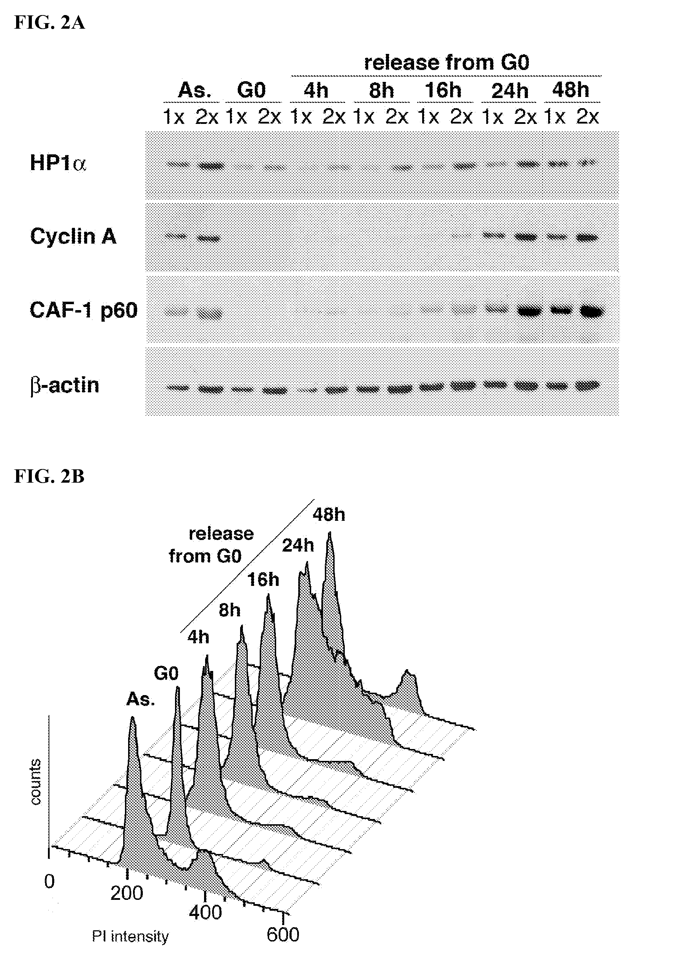

**Figure 2****: HP1**α **levels progressively increase after exit from quiescence.** ***Fig 2A******.* BJ** primary fibroblasts are arrested in quiescence (G0) by serum starvation and released by adding back serum for the indicated time period. Total protein levels are analyzed by Western blot. CAF-1 p60 (Polo et al, 2004) and Cyclin A are used as markers for cell proliferation. β-action is used as a loading control. ***Fig 2B******.*** Flow cytometry analysis of the cell cycle distribution of the cells shown in Fig 2A.

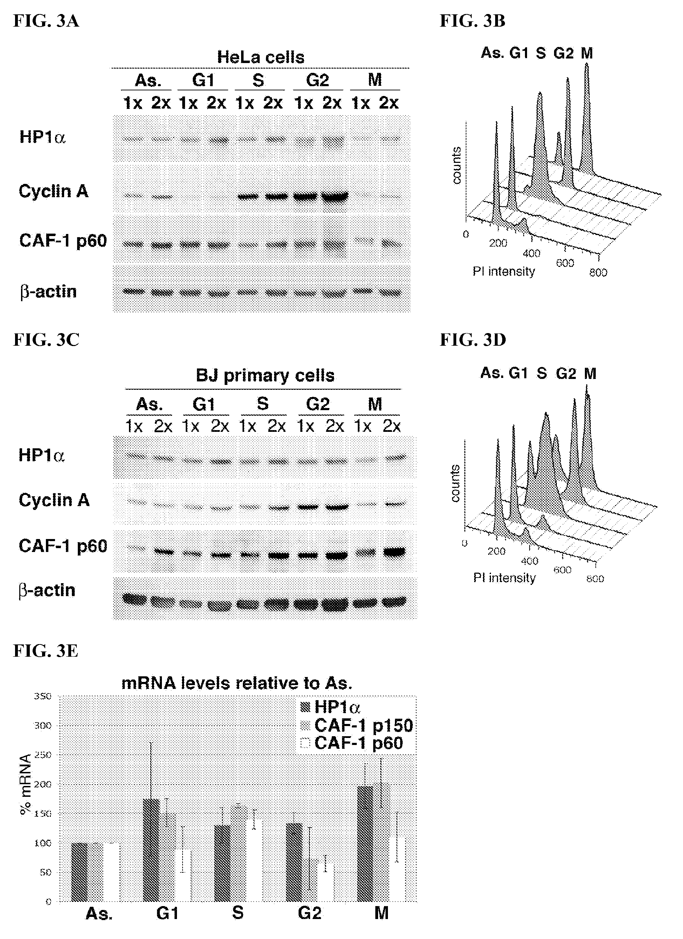

***Figure* 3****: *HP1***α ***expression does not visibly vary during the cell cycle.*** ***Fig 3A******.*** HeLa cervical carcinoma cells are synchronized in the cell cycle by release from double thymidine block (G1, G1/S, S, G2) and by nocodazole (M). Total protein extracts are analyzed for HP1α expression levels using Western Blot. β-actin is used as a loading control and Cyclin A as a marker for G2/S phases. CAF-1 p60 is shown for comparison. ***Fig 3B******.*** Flow cytometry cell cycle analysis of the synchronized cells used in Fig 3A. ***Fig 3C******.*** BJ foreskin primary fibroblasts are synchronized in the cell cycle by release from double thymidine block (G1, G1/S, S, G2) and by nocodazole (M). HP1α protein levels are analyzed in total cell extracts by Western Blot. β-actin is used as a loading control and Cyclin A as a marker for G2/S phases. CAF-1 p60 is shown for comparison. ***Fig 3D******.*** Flow cytometry analysis of the cell cycle distribution of the synchronized cells shown in Fig 3C. ***Fig 3E****.* HP1α mRNA levels in synchronized BJ cells, as determined by quantitative RT-PCR. Levels are normalized to the reference gene ribosomal protein P0-like protein (RPLPO) (de Cremoux et al, 2004) and levels in asynchronously proliferating cells are set to 100%. CAF-1 p60 and CAF-1 p150 levels are shown for comparison (Polo et al, 2004). Error bars represent data from two independent experiments.

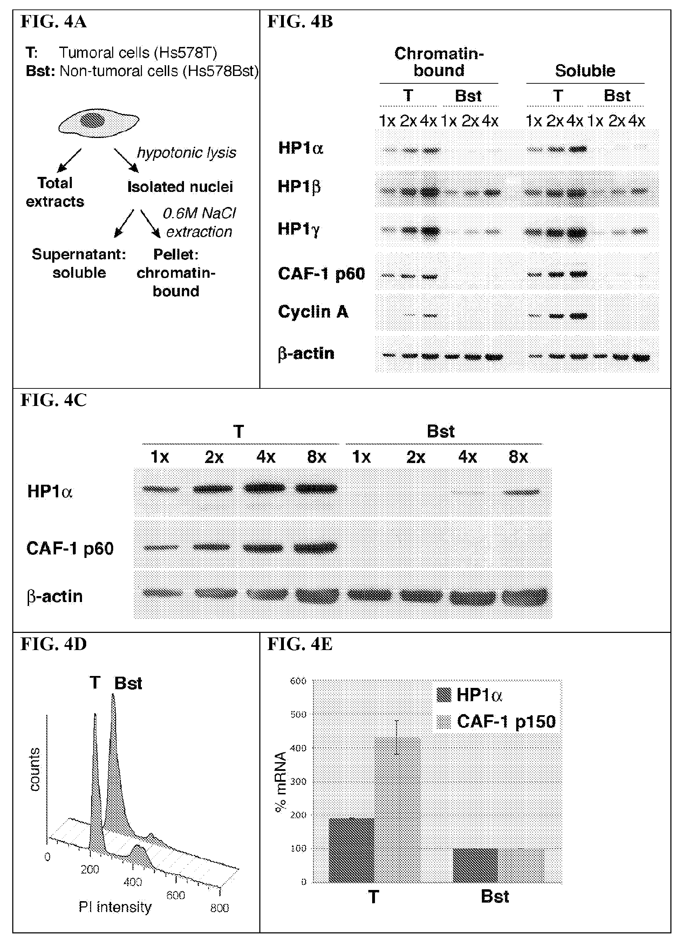

**Figure 4****: HP1**α **is overexpressed in breast cancer cells and associated with chromatin.** ***Fig. 4A******.*** Scheme of the experimental procedure applied to obtain total, soluble and chromatin-bound cell extracts as used in Fig. 4B. ***Fig. 4B******.*** HP1α, β and γ protein levels are analyzed by Western Blot in soluble and chromatin-bound nuclear extracts from the breast cancer cell line Hs578T (T) and in the non-tumoral mammary cell line Hs578Bst (Bst), which are derived from the same patient (Hackett et al, 1977). Cyclin A and CAF-1 p60 are shown for comparison. Increasing amounts (x) of total cells extracts are loaded; β-actin serves as a loading control. ***Fig. 4C****.* Relative quantification of total HP1α protein levels in tumoral (T) and non-tumoral (Bst) mammary cells, compared to the amounts of CAF-1 p60 (Polo et al, 2004). Increasing amounts (x) of total cells extracts are loaded; β-actin serves as a loading control. ***Fig. 4**D*. Flow cytometry analysis of tumoral (T) and non-tumoral (Bst) mammary cells in order to assess polyploidy. Tumoral (T) and non-tumoral (Bst) cells contain 25% and 13% of cells in S-phase, respectively. ***Fig. 4E***. Relative HP1α mRNA levels in tumoral (T) and non-tumoral (Bst) mammary cells, as determined by quantitative RT-PCR. Levels are normalized to the reference gene ribosomal protein P0-like protein (RPLPO) (de Cremoux et al, 2004) and non-tumoral cells are set to 100%. CAF-1 p150 is shown for comparison. Error bar represents data from two independent experiments.

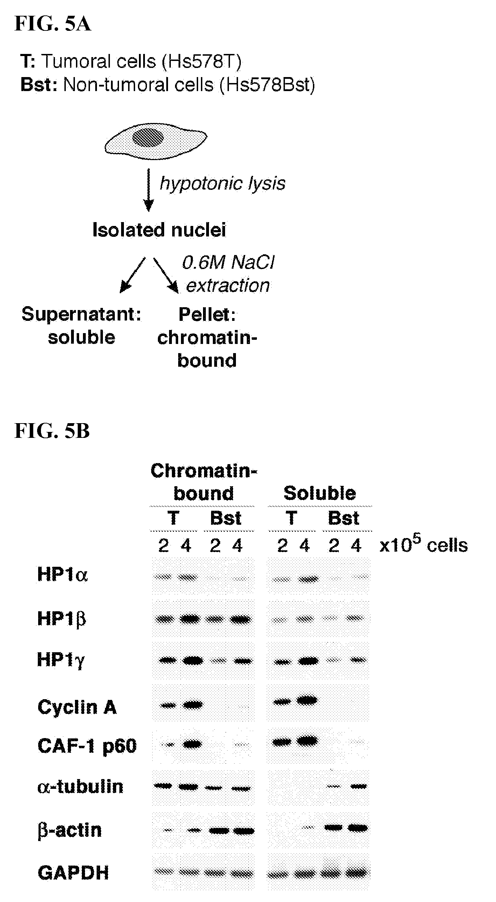

**Figure 5****: Levels of HP1 proteins in soluble and chromatin-bound nuclear fractions.** ***Fig. 5A*****.** Scheme depicting the extraction procedure to obtain soluble and chromatin-bound nuclear fractions of the breast cancer cell line Hs578T (T) and the healthy mammary cell line Hs578Bst (Bst), which are derived from the same patient (Hackett et al, 1977). ***Fig. 5B******.*** HP1α, β and γ protein levels are analyzed by Western Blot in soluble and chromatin-bound nuclear extracts from the cancer cells Hs578T (T) and the healthy cells Hs578Bst (Bst). Loading was corrected in a manner to deposit an identical amount of cells (2x105 and 4x105 cells for each extract). Several classical loading controls were revealed. While the fractionation of α-tubulin and the presence of β-actin differ slightly between tumoral and healthy cells, GAPDH reflects best the identical loading under these experimental conditions. Cyclin A and CAF-1 p60 are shown for comparison.

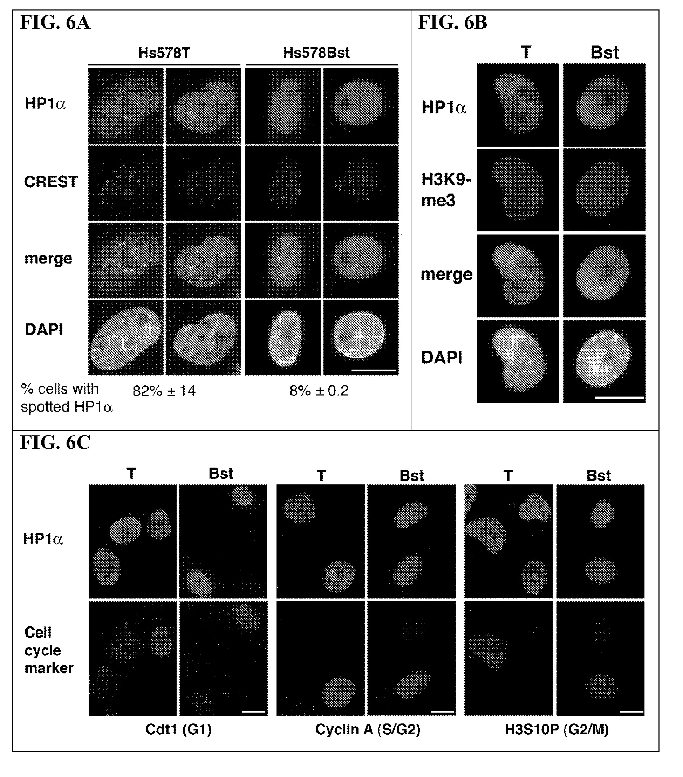

**Figure 6****: Nuclear distribution of HP1 α, but not H3K9me3, is altered in breast cancer cells compared to healthy mammary cells.** ***Fig 6A*****.** Immunofluorescence staining of HP1α in the breast cancer cell line Hs578T (T) and in the non-tumoral mammary cell line Hs578Bst (Bst). Costaining for centromeres, using CREST patient serum, reveals a partial although incomplete colocalization with the HP1α spots in Hs578T breast cancer cells. DNA is stained with DAPI. Indicated below the microscopy images are the mean percentages of cells showing clearly distinguishable HP1α spots in the breast cancer cell line Hs578T (T) and in the healthy mammary cell line Hs578Bst (Bst). Standard error, based on two independent experiments, is indicated. ***Fig. 6B******.*** Nuclear distribution of the H3K9me3 histone modification, compared to HP1α in the tumoral (T) and non-tumoral (Bst) mammary cells. DNA is revealed with DAPI. ***Fig. 6C***. Costaining of HP1α with Cdt1 (indicative for G1 phase), Cyclin A (indicative for S and G2 phase), or with histone H3 phosphorylated on S10 (H3S10P, indicative for G2 and M phase), does not reveal a correlation between cell cycle status and spotted HP1α patterns. Scale bars: 10 µm

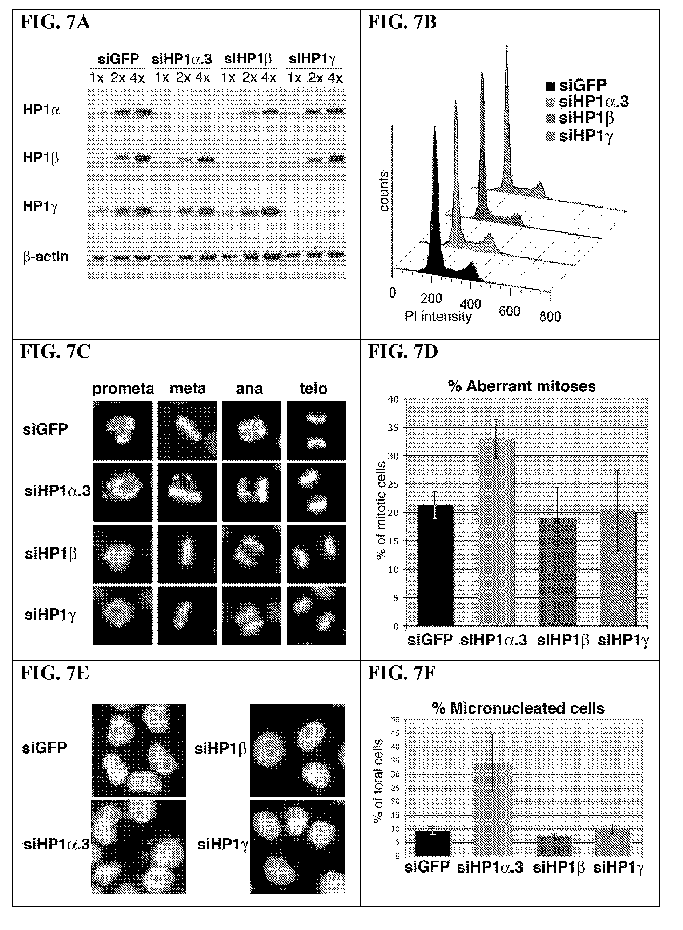

**Figure 7****: Downregulation of HP1**α**, but not HP1**β **or** γ**, leads to mitotic defects in HeLa cells.** ***Fig. 7A*****.** Western Blot analysis of total cell extracts from HeLa cells 72 hours after transfection with siRNA sequences targeting HP1α (named siHP1α.3), HP1β, HP1γ or control siRNA (siGFP). Increasing amounts (x) of total cells extracts are loaded and β-actin is used as a loading control. ***Fig. 7B******.*** Flow cytometry analysis of the cell cycle distribution of cells shown in *A.* ***Fig. 7C***. Typical mitotic figures after transfection with siRNA sequences targeting HP1α, HP1β, HP1γ or mock siRNA (siGFP). ***Fig. 7D****.* Percentage of aberrant mitotic figures (lagging chromosomes, misalignments, chromosome bridges) in asynchronous HeLa cells 72h after transfection with siRNA sequences targeting HP1α, HP1β, HP1γ or mock siRNA (siGFP). Error bars represent data from three independent, blind counted, experiments. ***Fig. 7E****.* DAPI staining reveals micronuclei formation 72h after transfection with an siRNA sequence targeting HP1α. ***Fig. 7F****.* Quantification of the percentage of micronucleated cells 72h after transfection with siRNA sequences targeting HP1α, HP1β, HP1γ or control siRNA (siGFP). Error bars represent data from three independent experiments of which two were blind counted.

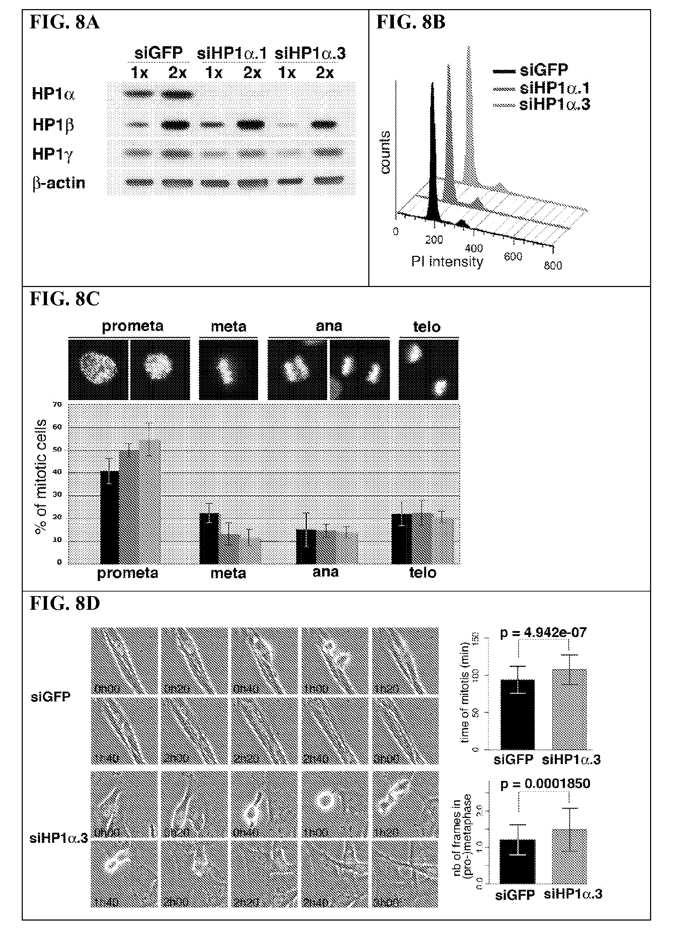

**Figure 8****: Downregulation of HP1α in primary cells affects (pro)metaphase.** ***Fig. 8A*****.** BJ primary fibroblasts are transfected by nucleofection with control siRNA (siGFP) or two different siRNA sequences targeting HP1α (α.1 or α.3). Total protein levels are analyzed by Western blot. HP1β and HP1γ are revealed to show the specificity of the siRNA for the HP1α isoform. ***Fig. 8B******.*** Flow cytometry analysis of the cell cycle distribution of cells shown in Fig. 8A, 72 hours after transfection with control siRNA (siGFP) or two different siRNA sequences targeting HP1α (siHP1α.1/3). ***Fig. 8C*****.** Profiles of mitotic cells are analyzed by immunofluorescence 72h after transfection. Enrichment in mitotic cells is achieved by a 6 hours release from a 12h thymidine block. Depicted are typical mitotic profiles (upper panel) and the presence of each mitotic phase as a percentage of the total number of mitotic cells (graph). Error bars represent standard errors, based on three independent experiments (n>100 for each) of which two were blind counted. ***Fig. 8D****.* Time of mitosis after two subsequent transfections with control siRNA (siGFP) or HP1α siRNA (siHP1α.3), as determined by live cell imaging. DNA is visualized by expression of H2B-Cherry (red). Images were acquired every 20 minutes for a period of 24h, starting 48h after the second transfection. The time of mitosis (upper graph) was defined as the time between the condensation of DNA and the establishment of two distinct cells. The average number of images depicting mitotic cells in (pro-)metaphase, before the onset if chromosome segregation, was also determined (lower graph). Means of three independent experiments are depicted, representing a total number of 130 and 87 mitotic cells for siGFP and siHP1α, respectively. P-values were determined with a test of Mann-Whitney Wilcoxon. Mean H2B-cherry transfection efficiencies were 37% and 39% for siGFP and siHP1α, respectively.

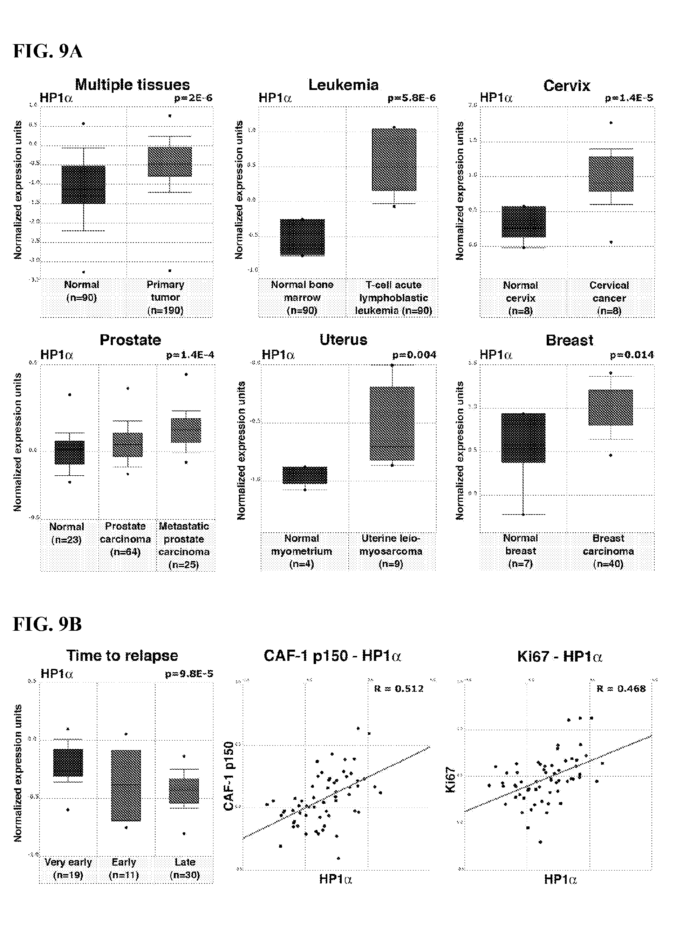

**Figure 9****: HP1α mRNA is overexpressed in multiple types of cancer and correlates with clinical and molecular data.** ***Fig. 9A******.*** Boxplot representation of microarray expression profiles of HP1α in different types of cancer compared to normal tissue. Boxes represent the 25th-75th percentile; brackets: range; black line: median; black dots: outliers; n: sample number. p-values are based on Student's T-test. Results were analyzed and plotted using ONCOMINE (Rhodes et al, 2004), and exploited data from transcriptome studies on different tumor types (Andersson et al, 2007; Pyeon et al, 2007; Quade et al, 2004; Ramaswamy et al, 2003; Richardson et al, 2006; Yu et al, 2004). ***Fig. 9B******.*** Left panel: Correlation of HP1α expression levels with the time to relapse in childhood acute lymphoblastic leukemia (Kirschner-Schwabe et al, 2006). Relapse occurs very early (within 18 months after initial diagnosis), early (>18 months after initial diagnosis but <6 months after cessation of frontline treatment) or late (>6 months after cessation of frontline treatment). Boxplot representation as in Fig. 9A. Right panels: Scatter plot representation of the positive correlation of HP1α expression levels with CAF-1 p150 and the proliferation marker Ki67 expression levels in childhood acute lymphoblastic leukemia (Kirschner-Schwabe et al, 2006). Grey line: line of best fit; R: Pearson's correlation coefficient. Results were analyzed and plotted using ONCOMINE (Rhodes et al, 2004).

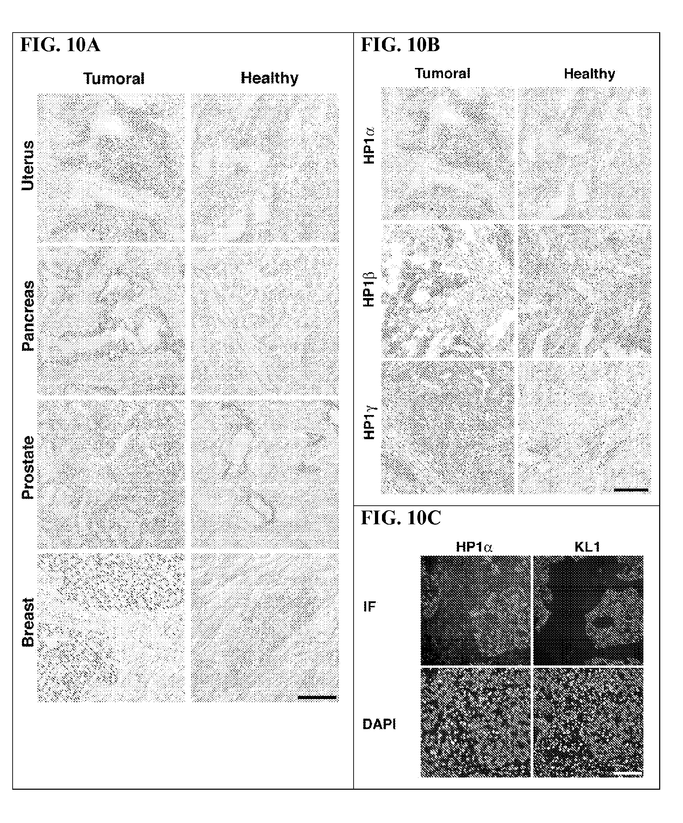

**Figure 10****: HP1**α **protein is overexpressed in multiple types of human cancer.** ***Fig. 10A*****.** Frozen human tissue arrays containing sections from tumoral and healthy origin were stained for HP1α by immunohistochemistry and counterstained with hematoxylin. ***Fig. 10B******.*** Frozen uterus tissue sections were stained for HP1α, β and γ by immunohistochemistry and counterstained with hematoxylin. Tissue arrays were processed identically for the three antibodies. ***Fig. 10C******.*** Two adjacent sections from a frozen medullary breast carcinoma were stained for HP1α and epithelial pan-cytokeratin marker KL-1, by immunofluorescence (IF). DNA is revealed with DAPI. Areas of HP1α overexpression correspond to regions of epithelial tumor cells. Scale bars: 100 µm.

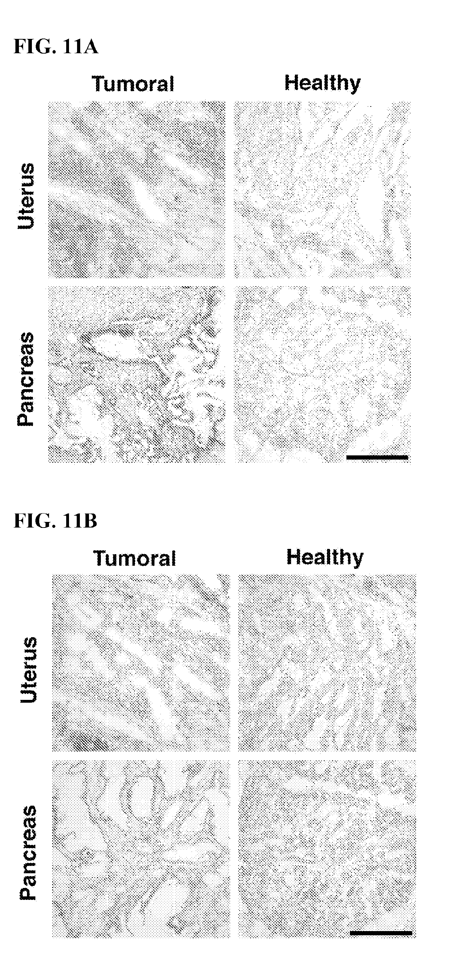

**Figure 11****: Overexpression of HP1**α **in human tumor samples is detected with a polyclonal antibody and is not accompanied by altered H3K9me3 staining.** ***Fig. 11A******.*** Immunohistochemistry staining, using a polyclonal HP1α antibody, of a tissue array containing frozen human tissue sections from tumoral and healthy origin. Tissues were counterstained with hematoxylin. ***Fig. 11B******.*** A frozen human tissue array (same batch as in Fig. 11A.) was stained for H3K9me3 by immunohistochemistry and counterstained with hematoxylin. Scale bars: 50 µm.

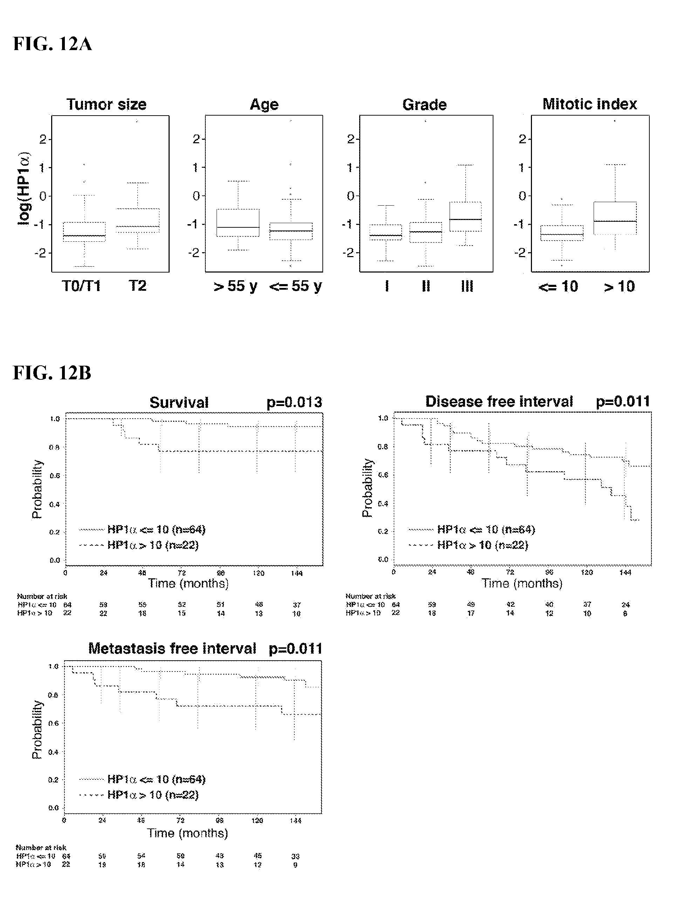

**Figure 12****: HP1α expression levels have a prognostic value in breast cancer patients.** ***Fig. 12A******.*** Boxplots representing logarithmic HP1α mRNA expression levels, according to the indicated clinico-pathological factors, in 86 breast cancer samples with a >10 year patient follow-up. Boxes represent the 25th-75th percentile; brackets: range; black line: median; black dots: outliers. ***Fig. 12B******.*** Univariate Kaplan-Meier curves of the survival, the occurrence of metastasis and the disease-free interval (interval before the occurrence of local recurrence, regional lymph node recurrence, controlateral breast cancer or metastasis) in patients expressing high (>10) or low (≤10) levels of HP1α. The number of patients at risk at each time point is indicated below the graphs.

### DETAILED DESCRIPTION OF THE INVENTION

Mammalian cells contain three closely related Heterochromatin Protein 1 isoforms, HP1α, β and γ, which, by analogy to their unique counterpart in *S.pombe*, have been implicated in gene silencing, genome stability and chromosome segregation.

A first possible link between HP1 proteins and tumorigenesis was put forward through the observation that HP1 interacts with the tumor suppressor Retinoblastoma protein (Rb) (Nielsen et al, 2001b; Williams & Grafi, 2000) and participates in the Rb-dependent silencing of cell cycle genes, such as Cyclin E (Nielsen et al, 2001b). Similarly, HP1 interacts with the transcriptional co-repressor KAP-1 (Ryan et al, 1999), which is involved in the regulation of the E2F1 (Wang et al, 2007) and p53 (Wang et al, 2005) proteins. Furthermore, HP1α and γ are found in a complex with Chromatin assembly factor 1 (CAF-1) (Murzina et al, 1999; Quivy et al, 2004), of which the intermediate subunit p60 is a validated proliferation marker in breast cancer (Polo et al, 2004). However, the specific and/or common regulation patterns of the three HP1 isoforms in relation to cell proliferation, quiescence and cancer remained elusive.

The inventors demonstrated that HP1α shows the unique property of displaying a proliferation-dependent expression pattern and that a significant overexpression of HP1α, but not β or γ, was observed in cancer cells when compared to non-tumoral cells derived from the same patient. This HP1α overexpression was observed in pancreas, uterus, ovary, prostate and breast carcinomas. These results show that HP1α constitutes a marker for diagnosis of multiple types of cancer. Inventors herein also demonstrate that HP1α expression levels in carcinomas correlate with clinical data and disease outcome. They show that high HP1α expression levels in carcinomas correlate with decreased patient survival, early disease progression, increased disease recurrence and/or increased occurrence of metastasis over time, i.e. a poor prognosis.

### Definitions

As used herein, the term "HP1α" refers to the heterochromatin protein 1-alpha, a highly conserved nonhistone protein. Three accession numbers correspond to the human HP1α in Genbank: NP_001120793, NP_001120794, NP_036249. This protein is encoded by the gene *CBX5* (GeneID: 23468).

The term "cancer" or "tumor", as used herein, refers to the presence of cells possessing characteristics typical of cancer-causing cells, such as uncontrolled proliferation, immortality, metastatic potential, rapid growth and proliferation rate, and certain characteristic morphological features. This term refers to any type of malignancy (primary or metastases). Typical cancers are solid or hematopoietic cancers such as breast, stomach, oesophageal, sarcoma, ovarian, endometrium, bladder, cervix uteri, rectum, colon, lung or ORL cancers, paediatric tumours (neuroblastoma, glyoblastoma multiforme), lymphoma, leukaemia, myeloma, seminoma, Hodgkin and malignant hemopathies. Preferably, the cancer is selected from the group consisting of breast cancer, pancreas cancer, uterus and cervix cancers, ovary cancer, prostate cancer and leukemia. Preferably the cancer is a solid cancer, more preferably an early stage solid cancer without local or systemic invasion. Preferably, the solid cancer is breast cancer, more preferably an early stage breast cancer without local or systemic invasion.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

As used herein, the term "chemotherapeutic treatment" or "chemotherapy" refers to a cancer therapeutic treatment using chemical or biochemical substances, in particular using one or several antineoplastic agents.

The term "radiotherapeutic treatment" or "radiotherapy" is a term commonly used in the art to refer to multiple types of radiation therapy including internal and external radiation therapies or radioimmunotherapy, and the use of various types of radiations including X-rays, gamma rays, alpha particles, beta particles, photons, electrons, neutrons, radioisotopes, and other forms of ionizing radiations.

The term "adjuvant therapy", as used herein, refers to chemotherapy or radiotherapy given as additional treatment, usually after surgical resection of the primary tumor, in a patient affected with a cancer that is at risk of metastasizing and/or likely to recur. The aim of such an adjuvant treatment is to improve the prognosis.

As used herein, the term "poor prognosis" refers to a decreased patient survival and/or an early disease progression and/or an increased disease recurrence and/or an increased metastasis formation.

As used herein, the term "subject" or "patient" refers to an animal, preferably to a mammal, even more preferably to a human, including adult, child and human at the prenatal stage. However, the term "subject" can also refer to non-human animals, in particular mammals such as dogs, cats, horses, cows, pigs, sheeps and non-human primates, among others, that are in need of treatment.

The term "sample", as used herein, means any sample containing cells derived from a subject, preferably a sample which contains nucleic acids. Examples of such samples include fluids such as blood, plasma, saliva, urine and seminal fluid samples as well as biopsies, organs, tissues or cell samples. The sample may be treated prior to its use. The term "cancer sample" refers to any sample containing tumoral cells derived from a patient, preferably a sample which contains nucleic acids. Preferably, the sample contains only tumoral cells. The term "normal sample" refers to any sample which does not contain any tumoral cells.

The methods of the invention as disclosed below, may be *in vivo*, *ex vivo* or *in vitro* methods, preferably *in vitro* methods.

In previous studies, an increased expression of HP1α has been correlated with a decreased invasive potential among several breast cancer cell lines (Kirschmann et al., 2000; Norwood et al., 2006). Inventors herein surprisingly demonstrate that high expression levels of HP1α are on the contrary associated with a poor prognosis and thus with an increase risk of death.

Accordingly, the present invention concerns a method for predicting or monitoring clinical outcome of a subject affected with a cancer, wherein the method comprises the step of determining the expression level of HP1α in a cancer sample from said subject, a high expression level of HP1α being indicative of a poor prognosis.

In an embodiment, the method further comprises the step of providing a cancer sample from the subject.

The expression level of HP1α can be determined from a cancer sample by a variety of techniques. In an embodiment, the expression level of HP1α is determined by measuring the quantity of HP1α protein or HP1α mRNA.

In a particular embodiment, the expression level of HP1α is determined by measuring the quantity of HP1α protein. The quantity of HP1α protein may be measured by any methods known by the skilled person. Usually, these methods comprise contacting the sample with a binding partner capable of selectively interacting with the HP1α protein present in the sample. The binding partner is generally a polyclonal or monoclonal antibody, preferably monoclonal. Polyclonal and monoclonal antibodies anti-HP1α are commercially available. Examples of these marketed antibodies are the mouse monoclonal anti-HP1α 2HP-1H5 from Euromedex (directed against amino acids 67-119), the rabbit polyclonal anti-HP1α H2164 from Sigma (directed against amino acids 177-191) and the mouse monoclonal anti-HP1α 2HP-2G9 from Euromedex. The other antibodies used in the different methods to quantify the HP1α protein are well known by the skilled person and are commercially available. The quantity of HP1α protein may be measured by semi-quantitative Western blots, enzyme-labeled and mediated immunoassays, such as ELISAs, biotin/avidin type assays, radioimmunoassay, immunoelectrophoresis or immunoprecipitation. The protein expression level may be assessed by immunohistochemistry on a tissue section of the cancer sample (e.g. frozen or formalin-fixed paraffin embedded material). The reactions generally include revealing labels such as fluorescent, chemiluminescent, radioactive, enzymatic labels or dye molecules, or other methods for detecting the formation of a complex between the antigen and the antibody or antibodies reacted therewith. Preferably, the quantity of HP1α protein is measured by immunohistochemistry or semi-quantitative western-blot. HP1α detection by immunohistochemistry does not require an antigen-unmasking step, unlike the routinely used marker Ki-67, and thus speeds up the staining process.

In another embodiment, the expression level of HP1α is determined by measuring the quantity of HP1α mRNA. Methods for determining the quantity of mRNA are well known in the art. For example the nucleic acid contained in the sample (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). Preferably quantitative or semi-quantitative RT-PCR is preferred. Real-time quantitative or semi-quantitative RT-PCR is particularly advantageous. Preferably, primer pairs were designed in order to overlap an intron, so as to distinguish cDNA amplification from putative genomic contamination. An example of primer pair which may be used in this method is presented in the experimental section and is constituted by the primers of SEQ ID NO.8 and SEQ ID NO.9. Other primers may be easily designed by the skilled person. Other methods of Amplification include ligase chain reaction (LCR), transcription-mediated amplification (TMA), strand displacement amplification (SDA) and nucleic acid sequence based amplification (NASBA). Preferably, the quantity of HP1α mRNA is measured by quantitative or semi-quantitative RT-PCR or by real-time quantitative or semi-quantitative RT-PCR.

In an embodiment, the method further comprises the step of comparing the expression level of HP1α to a reference expression level.

In a particular embodiment, the reference expression level is the expression level of HP1α in a normal sample. The normal sample is a non-tumoral sample, preferably from the same tissue than the cancer sample. The normal sample may be obtained from the subject affected with the cancer or from another subject, preferably a normal or healthy subject, i.e. a subject who does not suffer from a cancer. Preferably, the normal sample is obtained from the same subject than the cancer sample. Expression levels obtained from cancer and normal samples may be normalized by using expression levels of proteins which are known to have stable expression such as RPLPO (acidic ribosomal phosphoprotein PO), TBP (TATA box binding protein), GAPDH (glyceraldehyde 3-phosphate dehydrogenase) or β-actin.

In another embodiment, the reference expression level is the expression level of a gene having a stable expression in different cancer samples. Such genes include for example, RPLPO, TBP, GAPDH or β-actin. Preferably, the reference expression level is the expression level of the RPLPO gene. The use of the human acidic ribosomal phosphoprotein PO (RPLPO) gene as reference was described in the article of de Cremoux et al. (de Cremoux et al., 2004). In a preferred embodiment, the quantity of HP1α mRNA is normalized according to the quantity of RPLPO mRNA. The quantity of RPLPO mRNA is used as reference quantity (i.e. 100%). The quantity of HP1α mRNA is expressed as a relative quantity with respect to the quantity of RPLPO mRNA.

In a further embodiment, the method further comprises the step of determining whether the expression level of HP1α is high compared to the reference expression level.

If the reference expression level is the expression level of HP1α in a normal sample, the expression level of HP1α in the cancer sample is considered as high if, after normalization, the level is at least 1.5-fold higher than the expression level in the normal sample. Preferably, the expression level of HP1α in the cancer sample is considered as high if the level is at least 2-fold higher, or 3, 4, 5 or 6-fold higher, than the expression level in the normal sample.

If the reference expression level is the expression level of a gene having a stable expression in different cancer samples, in particular the expression level of the RPLPO gene, the expression level of HP1α is considered as high if the level or quantity of HP1α mRNA is of at least 7, 8, 9, 10, 11, 12, 13, 14 or 15% of the RPLPO mRNA level or quantity. In a preferred embodiment the expression level of HP1α is considered as high if the level or quantity of HP1α mRNA is of at least 10% of the RPLPO mRNA level or quantity. This cut-off value may be easily adjusted by the skilled person using another reference gene. This cut-off value is in particular applicable for breast cancer. This value may vary depending on the type of cancer and will be easily adapted by the one skilled in the art.

In an embodiment, the present method further comprises assessing at least one another cancer or prognosis marker such as tumor grade, hormone receptor status, mitotic index, tumor size or expression of proliferation markers such as Ki67, MCM2, CAF-1 p60 and CAF-1 p150. These markers are commonly used and the results obtained with these markers may be combined with the results obtained with the present method in order to confirm the prognosis. The use of these markers is well-known by the skilled person. As example, the tumor grade may be determined according the Elston & Ellis method (Elston & Ellis, 2002), the hormone receptor status (estrogen and progesterone) may be determined at the protein or at the mRNA level, the mitotic index may be determined by counting mitotic cells in ten microscopic fields of a representative tissue section and the tumor size can be detected by imaging techniques (e.g. mammography), by palpation or after surgery in the excised tissue. Expression of Ki67 and CAF-1 can be assessed at the protein level or at the mRNA level. High grade, high mitotic index, large size and/or high Ki67 or CAF-1 expression are indicative for a worse prognosis.

Using multivariate statistical analyses, the inventors have demonstrated, in the experimental section below, that HP1α expression levels predict disease outcome better than these standard prognostic markers.

Until now, the expression level of HP1 has been compared in different cancer cell lines but no study was performed to compare the HP1 expression levels in tumoral and non-tumoral cells. The inventors herein show, for the first time, that HP1α is specifically overexpressed in tumoral cells when compared to the HP1α expression in non-tumoral cells derived from the same patient. Such an overexpression thus constitutes a marker for diagnosis of multiple types of cancer, in particular breast cancer.

Accordingly, the present invention concerns a method for diagnosing a cancer in a subject, wherein the method comprises the step of determining the expression level of HP1α in a sample from said subject, a high expression level of HP1α indicating that said subject suffers from a cancer.

In an embodiment, the method further comprises the step of providing a sample from the subject. Preferably, this sample is suspected to contain tumoral cells.

The expression level of HP1α in this sample is determined as described above, preferably by measuring the quantity of HP1α protein or HP1α mRNA.

In an embodiment, the method further comprises the step of comparing the expression level of HP1α to a reference expression level, preferably, the expression level of HP1α in a normal sample. The normal sample is, as described above, a non-tumoral sample, preferably from the same tissue than the sample to be tested. The normal sample may be obtained from the subject to be diagnosed or from another subject, preferably a healthy subject. Expression levels obtained from the normal sample and from the sample to be tested may be normalized using expression levels of protein which are known to have a stable expression, such as described herein above.

In a further embodiment, the method further comprises the step of determining whether the expression level of HP1α is high compared to said reference expression level. A subject is diagnosed to suffer from a cancer if the expression level of HP1α in the sample to be tested is at least 10, 25, 50 or 75% higher than in the normal sample. Preferably, the subject is diagnosed to suffer from a cancer if the expression level of HP1α in the sample to be tested is at least 25% higher than in the normal sample.

In another embodiment, the present method further comprises assessing at least one another marker used to diagnose cancer such as tumor grade, mitotic index, tumor size or expression of proliferation markers such as Ki67, MCM2, CAF-1 p60 and CAF-1 p150. These markers are commonly used for diagnostic purposes and the results obtained with these markers may be combined with the results obtained with the present method in order to confirm the diagnosis.

The present invention also concerns a method for providing useful information for the diagnosis of a cancer in a subject, wherein the method comprises the step of determining the expression level of HP1α in a sample from said subject, a high expression level of HP1α indicating that said subject suffers from a cancer. In an embodiment, the method further comprises the step of providing a cancer sample from the subject.

In a further aspect, the present invention concerns a method for selecting a subject affected with a cancer for an adjuvant chemotherapy and/or radiotherapy or determining whether a subject affected with a cancer is susceptible to benefit from an adjuvant chemotherapy and/or radiotherapy, wherein the method comprises the step of determining the expression level of HP1α in a cancer sample from said subject, a high expression level of HP1α indicating that an adjuvant chemotherapy and/or radiotherapy is required.

In an embodiment, the method further comprises the step of providing a cancer sample from the subject.

The expression level of HP1α in the cancer sample is determined as described above, preferably by measuring the quantity of HP1α protein or HP1α mRNA.

In an embodiment, the method comprises the step of comparing the reference expression level of HP1α to a reference expression level as described above in relation with the method for predicting clinical outcome. The reference expression level may be the expression level of HP1α in a normal sample or the expression level of a gene having a stable expression in different cancer samples, such as the RPLPO gene.

In a further embodiment, the method further comprises the step of determining whether the expression level of HP1α is high compared to said reference expression level. In a particular embodiment, the expression level of HP1α in the cancer sample is considered as high if, after normalization, the level is at least 1.5, 2, 3, 4, 5 or 6-fold higher than the expression level in the normal sample. Preferably, the expression level of HP1α in the cancer sample is considered as high if the level is at least 1.5-fold higher than the expression level in the normal sample. In another particular embodiment, the expression level of HP1α is considered as high if the quantity of HP1α mRNA is of at least 7, 8, 9, 10, 11, 12, 13, 14 or 15% of the RPLPO mRNA quantity. Preferably, the expression level of HP1α is considered as high if the quantity of HP1α mRNA is of at least 10% of the RPLPO mRNA quantity.

As explained above, these cut-off values are particularly adapted to breast cancer and may vary according to the type of cancer. However, the skilled person can easily adapt this cut-off.

A high expression level of HP1α indicates a decreased patient survival and/or an early disease progression and/or an increase disease recurrence and/or an increase metastasis formation. Accordingly, this type of cancer associated with poor prognosis has to be treated with adjuvant chemotherapy and/or radiotherapy in order to improve the patient's chance for survival. The type of adjuvant therapy is chosen by the practitioner.

In an embodiment, the present method further comprises assessing at least one another cancer and prognosis marker such as tumor grade, hormone receptor status, mitotic index, tumor size or expression ofproliferation markers such as Ki67, MCM2, CAF-1 p60 and CAF-1 p150. The results obtained with these markers may be used to confirm the result obtained with the method according to the invention and/or to orientate the choice of the adjuvant therapy.

In a last aspect, the present invention further concerns a method for monitoring the response to a treatment of a subject affected with a cancer, wherein the method comprises the step of determining the expression level of HP1α in a cancer sample from said subject before the administration of the treatment, and in a cancer sample from said subject after the administration of the treatment, a decreased expression level of HP1α in the sample obtained after the administration of the treatment indicating that the subject is responsive to the treatment.

In an embodiment, the method further comprises the step of providing a cancer sample from the subject.

The treatment may be any chemotherapeutic or radiotherapeutic treatment. A first cancer sample is obtained from the subject before the administration of the treatment. A second sample is obtained from the same subject after the administration of the treatment. Preferably, the second sample is collected when a significant effect on cell proliferation can be expected, dependent on the chosen treatment. In an embodiment, the second sample is collected at least 2 days after administration of the treatment, preferably one week after said administration.

The responsiveness of the patient to the treatment is evaluated by determining the expression level of HP1α in tumoral cells before and after the treatment. The expression level of HP1α in the cancer sample is determined as described above, preferably by measuring the quantity of HP1α protein or HP1α mRNA. A lower expression level of HP1α in tumoral cells contained in the sample collected after the treatment in comparison with the expression level of HP1α in tumoral cells contained in the sample collected before the treatment indicates that the subject is responsive to the treatment.

In an embodiment, the method further comprises assessing at least one another cancer marker such as tumor grade, hormone receptor status, mitotic index, tumor size or expression of proliferation markers such as Ki67, MCM2, CAF-1 p60 and CAF-1 p150.

This method may also provide an indication of the required duration and/or intensity of the treatment. In this case, a follow-up after each cycle of treatment permits to determine if the expression level of HP1α is lower than before the treatment, and thus to adjust the treatment duration and/or intensity accordingly.

The present invention further concerns the use of HP1α as a prognosis marker in cancer, preferably in human cancer and more preferably in human breast cancer. In a preferred embodiment, HP1α is used as a prognosis marker in an early stage breast cancer without local or systemic invasion. As used herein, the term "prognosis marker" refers to a compound, i.e. HP1α, used to predict or monitor clinical outcome of a subject affected with a cancer.

The present invention also concerns the use of HP1α as a diagnosis marker for cancer, preferably for human cancer and more preferably for human breast cancer. As used herein, the term "diagnosis marker" refers to a compound, i.e. HP1α, used to diagnose a cancer in a subject.

The present invention also concerns the use of HP1α as a marker for selecting a subject affected with a cancer for an adjuvant therapy and/or radiotherapy or determining whether a subject affected with a cancer is susceptible to benefit from an adjuvant chemotherapy and/or radiotherapy. Preferably, the cancer is a human cancer and more preferably for human breast cancer.

The present invention further concerns the use of HP1α as a marker for monitoring the response to a treatment of a subject affected with a cancer. Preferably, the cancer is a human cancer and more preferably for human breast cancer.

HP1α may be used as marker in any one of the methods of the invention as described above.

In another aspect, the present invention further concerns a kit
(a) for predicting or monitoring clinical outcome of a subject affected with a cancer; and/or
(b) for diagnosing a cancer in a subject; and/or
(c) for selecting a subject affected with a cancer for an adjuvant therapy and/or radiotherapy or determining whether a subject affected with a cancer is susceptible to benefit from an adjuvant chemotherapy and/or radiotherapy; and/or
(d) for monitoring the response to a treatment of a subject affected with a cancer, wherein the kit comprises
   (i) at least one antibody specific to HP1α and, optionally, means for detecting the formation of the complex between HP1α and said at least one antibody; and/or
   (ii) at least one probe specific to the protein HP1α and, optionally, means for detecting the hybridization of said at least one probe on HP1α protein; and/or
   (iii) at least one nucleic acid primer pair specific to HP1α mRNA and, optionally, means for amplifying and/or detecting said mRNA; and,
   (iv) optionally, a leaflet providing guidelines to use such a kit.

Further aspects and advantages of the present invention will be described in the following examples, which should be regarded as illustrative and not limiting.

### EXAMPLES

### MATERIALS AND METHODS

### Cell culture and synchronization

Wi38 primary lung fibroblasts (ATCC) and BJ primary foreskin fibroblasts (ATCC) were cultured in MEMα medium (GIBCO), MCF7 breast cancer cells and HeLa cervical carcinoma cells in DMEM (GIBCO), Hs578T breast cancer cells in RPMI medium (GIBCO) containing 10mM insulin (Sigma) and Hs578Bst healthy mammary cells (ATCC) in DMEM containing 30 ng/ml Epidermal Growth Factor (TEBU). Media were supplemented with 10% fetal calf serum (Eurobio) and 10mg/ml penicillin and streptomycin (GIBCO).

For synchronization in quiescence, primary cells were grown in serum-free medium for at least 72h and MCF7 cells for 48h in medium containing 10nM of the anti-estrogen ICI182780 (Fischer Bioblock Scientific) (Carroll et al, 2000).

Synchronization was checked by flow cytometry as in (Polo et al, 2004) and data analyzed using FlowJo (Tree Star Inc.).

HeLa cells were synchronized in the different stages of the cell cycle as described in (Polo et al, 2004). BJ cells were synchronized similarly, except that they were blocked for 14h, released for 10h, blocked again for 14h and released for 3h, 6h or 12h for S-phase, G2 and G1, respectively. For mitotic BJ cells, 10ng/ml nocodozole was added 4h after the second release from thymidine and cells were harvested 10h later. Synchronization was analyzed by flow cytometry as described in (Polo et al, 2004). Analysis of flow cytometry data was done using FlowJo (Tree Star Inc.) software.

### Transfections and siRNAs

HeLa cells, plated in medium without antibiotics, were transfected with 30nM siRNA using Oligofectamine reagent (Invitrogen) and Optimem 1 medium (Gibco). siRNA sequences: siGFP: AAGCUGGAGUACAACUACAAC (SEQ ID No.1); siHP1α_1: CCUGAGAAAAACUUGGAUUTT (SEQ ID No.2) (Obuse et al, 2004); siHP1α_3: GGGAGAAGTCAGAAAGTAA (SEQ ID No.3); siHP1β: AGGAATATGTGGTGGAAAA (SEQ ID No.4); siHP1γ: AGGTCTTGATCCTGAAAGA (SEQ ID No.5).

BJ primary cells were transfected at passage 27 (8 passages after reception from ATCC) using nucleofection (Amaxa), according to manufacturer's instructions. Briefly, 4x10⁵ cells were transfected with 0.8 nmol siRNA (Dharmacon) or 1 µg of plasmid DNA in 100ul Nucleofector solution R (Amaxa), using the nucleofection program X-001. Cells were subsequently plated at a density of 7000 cells/cm2 to permit cell proliferation without reaching confluency within 4 days.

### Live cell imaging

For live cell imaging, BJ cells were transfected twice with siRNA, with a 72h interval. A plasmid coding for H2B-cherry was introduced in the second transfection, and cells were plated on glass bottom dishes (Mattek). Movies were made using the BioStation system (Nikon). Images were acquired every 20 minutes, during a period of 24 hours, starting 48 hours after the second transfection. Movies were analyzed using Image J.

### Cell extracts

For total cell extracts, after a wash in PBS, cells were scraped from plates in 1x Laemmli buffer (60 mM Tris-Hcl pH 6.8 ; 10% glycerol; 2% sodium dodecyl sulfate (SDS) ; 1% 2-mercaptoethanol and 0.002% bromophenol blue) and these extracts were boiled 10 min before storage at -20°C.

Chromatin-bound cell extracts were made according to (Martini et al, 1998). Protein concentrations were determined using Bio-Rad Protein Assay solution.

### Antibodies

Primary antibodies: mouse monoclonal anti-HP1α 2HP-1H5 from Euromedex and rabbit polyclonal anti-HP1α H2164 from Sigma (directed against amino acids 67-119 and 177-191, respectively) for immunofluorescence and immunohistochemistry, mouse monoclonal anti-HP1α 2HP-2G9 for Western blot (Euromedex), mouse monoclonal anti-HP1β lMODlA9 (Euromedex), mouse monoclonal anti-HP1γ2MOD-1G6-AS (Euromedex), anti-CREST human serum, rabbit polyclonal anti-CAF-1 p60, mouse monoclonal anti-β-actin AC-15 (Sigma), mouse monoclonal anti-KLl antibody (Dako), rabbit polyclonal anti-Cyclin A (Santa Cruz), mouse monoclonal anti-H3S10P (Abcam ab14955) and rabbit polyclonal anti-Cdtl (Santa Cruz 28262)..

Secondary antibodies: Fluorescein isothiocyanate (FITC) or Texas Red-coupled antibodies (immunofluorescence) or horseradish peroxidase-coupled antibodies (Western Blotting) were from Interchim.

### Western Blotting

After 30min treatment with 25U benzonase nuclease (Novagen), cell extracts were loaded on 4-12% gradient gels (Invitrogen), using Ix MES migration buffer (Invitrogen), followed by Western Blotting procedures as in (Martini et al, 1998).

### Immunofluorescence

After fixation in 2% paraformaldehyde, cells were permeabilized in 0.2% Triton X-100 in PBS. Immunofluorescence detection was carried out as in (Martini et al, 1998). Coverslips were mounted in Vectashield mounting medium containing 4',6-Diamidino-2-phenylindole (DAPI) (Vector Laboratories).

For immunofluorescence on tissue samples, 8 µm cryosections made from frozen mammary tissues (Curie Institute, Paris, France) were fixed on glass slides in 3% paraformaldehyde and immunostained as above.

### Immunohistochemistry

8 µm cryosections from frozen mammary tissues (Curie Institute, Paris, France) or frozen tissue arrays (FMC401, Biomax) were used. Sections, fixed on glass slides in 3% paraformaldehyde and permeabilized in PBS containing 0.5% Triton for 4 min, were incubated 5 min in 3% H₂O₂ (Prolabo) for peroxidase inhibition and blocked in PBS containing 1% BSA and 5% non-fat milk. Incubation with primary antibody diluted in blocking solution was followed by revelation with horseradish peroxidase-coupled secondary antibody (DakoCytomation) and Diaminobenzidine (DakoCytomation) before counterstaining with hematoxyline (Merck). Slides were dehydrated in increasing ethanol concentrations and toluene before mounting in Entellan mounting medium (Merck).

### RNA extraction, Quantitative RT-PCR and primers

The mRNeasy mini kit (Qiagen) was used for total RNA extraction from cell lines or frozen patient samples and cDNA were produced using Superscript II reverse transcriptase (Invitrogen) with 1 µg RNA and 3 µg of random primers (Invitrogen) per reaction. The Lightcycler 2.0 System (Roche) and the Lightcycler FastStart DNA Master SYBR Green I reaction kit (Roche) were used for quantitative PCR. For the patient samples, the 96-well plate Step One Plus system (Applied Biosystems) and the SYBR Green PCR Master mix (Applied Biosystems) were used. Duplicates were measured and three subsequent cDNA dilutions were carried out to assess primer efficiency. Primer pairs were designed in order to overlap an intron, so as to distinguish cDNA amplification from putative genomic contamination. Primers: RPLPO forward: GGCGACCTGGAAGTCCAACT (SEQ ID NO.6); RPLPO reverse: CCATCAGCACCACAGCCTTC (SEQ ID NO.7); HP1α forward: GATCATTGGGGCAACAGATT (SEQ ID NO.8); HP1α reverse: TGCAAGAACCAGGTCAGCTT (SEQ ID NO.9); CAF-1 p150 forward: CAGCAGTACCAGTCCCTTCC (SEQ ID NO.10); CAF-1 p150 reverse: TCTTTGCAGTCTGAGCTTGTTC (SEQ ID NO.11); CAF-1 p60 forward: CGGACACTCCACCAAGTTCT (SEQ ID N0.12); CAF-1 p60 reverse: CCAGGCGTCTCTGACTGAAT (SEQ ID NO.13). The quantity of HP1α mRNA was normalized according to the human acidic ribosomal phosphoprotein PO (RPLPO) (de Cremoux et al, 2004) by applying x=E(Cp RPLPO - Cp HP1α), in which E was the mean efficiency of primer pairs and in which x reflected the quantity of HP1α mRNA relative to the quantity of RPLPO mRNA in a given sample.

### Breast cancer patient samples and statistics

The present study included 92 breast cancer samples, selected from the Institut Curie Biological Resources Center for treatment with primary conservative tumorectomy (median tumor size: 18mm [6-50mm]). Patients were diagnosed in 1995 and found to be lymph node negative (N0) and metastasis free (M0). Patients were informed of research purposes and did not express opposition. Patient's and tumor characteristics are provided in the above Table I. RNA was extracted from cryopreserved tissue and analyzed as described above. RNA of 86 samples was of sufficient quality for further analysis.

Differences between groups were analysed by χ 2 or Fisher exact tests for categorical variables and Kruskal-Wallis for continuous variables. Recurrence-free and alive patients were censored at the date of their last known contact. Survival data were defined as the time from diagnosis of breast cancer until the occurrence of disease progression, defined as local recurrence in the treated breast, regional recurrence in lymph node-bearing areas, controlateral breast cancer or distant recurrences. Determination of a cut-off value prognostic for the Disease free Interval (DFI) was computed using a Cox proportional risks model. A Wald test was used to evaluate the prognostic value of this variable on each event. The overall survival (OS), Metastasis Free Interval and DFI rates were estimated by the Kaplan-Meier method, and groups were compared using a log-rank test. Multivariate analysis was carried out to assess the relative influence of prognostic factors on OS and DFI, using the Cox stepwise forward procedure (Cox 1972). Significance level was 0.05. Analyses were performed using R software 2.5.0 version.

### RESULTS

### HP1α expression depends on cell proliferation

Recent studies have described a downregulation of HP1 proteins in differentiated blood cells compared to undifferentiated blood cells (Baxter et al, 2004; Gilbert et al, 2003; Istomina et al, 2003; Ritou et al, 2007). This downregulation could be a common response to cell cycle exit, or a specific consequence of (blood) cell differentiation. To address this issue, the inventors examined whether transient cell cycle exit, which is not accompanied by a differentiation process, similarly results in HP1 downregulation. Using two different human primary fibroblast cell lines (WI38 and BJ), in which quiescence is induced by serum starvation, the inventors observed lower protein levels of HP1α, but not β or γ, in quiescent cells compared to proliferating cells (Fig 1A). MCF7 breast carcinoma cells, which are arrested in quiescence by anti-estrogen treatment (Carroll et al, 2000), show a similar downregulation of HP1α, but not β or γ. Thus, using two different means to induce quiescence, the inventors find a specific downregulation of the HP1α isoform, the extent of which correlates to the time cells have spent in quiescence. As a control, they verified the downregulation of CAF-1 p60 in all quiescent cells (Polo et al, 2004) (Fig 1A) and assessed synchronization efficiency by flow cytometry (Fig 1B). Similarly to CAF-1 p150 and p60, HP1α downregulation in transient quiescence relates in part to transcriptional regulation, since quiescent BJ cells also show decreased HP1α mRNA levels when compared to asynchronously proliferating cells (determined by quantitative RT-PCR, Fig 1C). Upon exit from the quiescent state, HP1α protein levels gradually increase between 16 and 24 hours after release (Fig 2A), which corresponds to the moment of cell cycle entry (determined by flow cytometry, Fig 2B). The accumulation of HP1α occurs earlier than the one of CAF-1 p60 (Fig 2A), mostly observed at the onset of DNA replication.

The observed downregulation of HP1α in quiescence could either be specific for the quiescent state or reflect an expression restricted to a specific stage of the cell cycle. The inventors therefore analyzed HP1α levels during the cell cycle in synchronized HeLa cells. In contrast to the cell cycle marker Cyclin A, they did not observe significant variation in HP1α levels in the synchronized cell populations (Fig 3A, B). Similarly, human primary fibroblasts, which display a normal cell cycle regulation, show essentially similar levels of HP1α protein (Fig 3C) and mRNA in the synchronized cell population (Fig 3D, E). In this respect, HP1α behaves similarly to CAF-1 p60 and p150, which are also ubiquitously expressed during the cell cycle but downregulated in quiescence (Fig 1 and 3; (Polo et al, 2004)). In conclusion, HP 1α expression levels are high at all stages of the cell cycle in proliferative cells, and HP1α downregulation is specific for the quiescent state.

### HP1α is overexpressed in breast cancer cells

The proliferation-dependent expression of HP1α suggests a possible differential expression between tumoral and non-tumoral cells, as found for proliferation markers including CAF-1 p60 (Polo et al, 2004). To examine this issue, the inventors used mammary cells derived from the same patient, either tumoral (Hs578T) or non-tumoral (Hs578Bst) (Hackett et al, 1977), to be relevant in our comparison. Besides total cell extract, they analyzed levels of HP1 proteins both in the soluble fraction and in the fraction bound to chromatin (Fig 4A). Indeed, HP1 proteins distribute in different nuclear fractions, distinguished by their capacity to resist extraction with high salt concentrations or with Triton X-100 detergent. The salt- or detergent-resistant pool of HP1 is considered as the active, chromatin-bound pool of HP1 and represents less than 10% of total HP1 in human and rodent cells, as determined by Western Blot quantification (Taddei et al, 2001) or FRAP (Cheutin et al, 2003; Dialynas et al, 2007; Festenstein et al, 2003).

Both in the chromatin-bound and in the soluble nuclear fraction, the inventors observed higher HP1α levels in tumoral Hs578T cells than in non-tumoral Hs578Bst cells, while HP1α or γ display little differences in expression compared to the loading control β-actin (Fig 4B). Loading of identical amounts of cells also shows tumoral overexpression of HP1α, specifically (Fig 5). Semi-quantitative Western Blot on total cell extracts shows that tumoral Hs578T cells contain approximately 8 times more HP1α protein than non-tumoral Hs578Bst cells, compared to the loading control (Fig 4C). The inventors could exclude the possibility that HP1α expression levels reflect the DNA content of the cell, since the tumoral Hs578T cells display only a very moderate aneuploidy in flow cytometry (Fig 4D). The overexpression of HP1α mRNA, detected by quantitative RT-PCR (Fig 4E), further indicates a regulation that involves at least in part transcription.

The present analysis of chromatin-bound and soluble fractions suggests that overexpressed HP1α in tumoral cells is partially chromatin-bound and thus possibly important for chromatin organization. This seems consistent with its nuclear localization, which is granular and diffuse in the non-tumoral mammary cells but clearly localized into discrete spots in a large fraction of the breast cancer cells (Fig 6A). These spots largely localize to centromeric regions, detected by CREST autoimmune serum (Fig 6A). Yet, the different localization of HP1α was not accompanied by an altered nuclear distribution of H3K9me3 (Fig 6B) and the different patterns of HP1α staining did not seem associated with specific stages of the cell cycle, as detected by staining for cell cycle markers (Fig 6C). In conclusion, the present cell line model shows an overexpression of HP 1α, but not HP 1 β or γ, in tumoral mammary cells compared to non-tumoral mammary cells. A large fraction of HP1α in breast cancer cells is chromatin-bound and localizes to centromeric regions.

### HP1α downregulation results in mitotic defects

The proliferation-dependent expression of HP1α, which is not observed for HP1β or γ, points to a unique function of this isoform and suggests that high amounts of this protein could confer an advantage for cell growth. Interestingly, downregulation of CAF-1 p150 results in a pronounced S-phase arrest in a manner that depends on its interaction with HP1 proteins (Quivy et al, 2008; Quivy et al, 2004). The inventors thus tested whether high expression levels of HP1α, β or γ are required for human cancer cell proliferation. By transfecting HeLa cells with siRNA against HP1α, β or γ, they obtained a specific downregulation of each of the HP1 isoforms (Fig 7A). In contrast to downregulation of its binding partner CAF-1 p150 (Quivy et al, 2008), downregulation of the HP1 isoforms did not result in any obvious effect on cell proliferation, as assessed by flow cytometry (Fig 7B). The inventors obtained similar results for HP1α downregulation in the mammary carcinoma cell line Hs578T. However, in agreement with previous reports (Auth et al, 2006; Obuse et al, 2004), the inventors did observe an increased fraction of mitotic profiles displaying lagging chromosomes, misalignments and chromosome bridges (Fig 7C) after downregulation of HP1α. They quantified these as the fraction of aberrant mitoses (Fig 7D). Interestingly, under the present experimental conditions, downregulation ofHP1β or γ did not give rise to increased mitotic defects, suggesting that only the HP1α isoform is critical for faithful mitosis. Furthermore, defects in mitosis were supported by observation of a three-fold increase in micronuclei formation after downregulation of HP1α, but not HP1β or γ (Fig 7E, F).

Since HeLa cells already show a high fraction of deficient mitoses (∼20%) and of micronucleated cells (∼10%), the inventors used primary fibroblasts, proficient for cell cycle control and checkpoint activity for further analysis. Transient transfection with two different siRNAs against HP1α resulted in a specific downregulation of this isoform (Fig 8A). Again, the inventors did not detect any significant effect on global cell cycle distribution by flow cytometry (Fig 8B), but they observed a small but reproducible increase in the percentage of prometaphases and a decreased percentage of metaphases by microscopy (Fig 8C). Interestingly, this effect mimics observations upon mutation of the *Drososophila* homologue of HP1α (Kellum & Alberts, 1995) or inactivation of the centromeric histone H3 variant CENPA in chicken cells (Regnier et al, 2005). Using live cell imaging, they measured a small but statistically significant (p = 4.9e-7) increase in the duration of mitosis after downregulation of HP1α compared to control (upper graph Fig 8D). Interestingly, the delay again mostly affected steps preceding actual chromosome segregation (Fig 8D, lower graph). In conclusion, the present observations in both tumoral and primary cells suggest a role for HP1α in early mitosis, possibly contributing to the correct alignment or the stable attachment of chromosomes at the metaphase plate.

### HP1α overexpression in human cancer samples

The proliferation-dependent expression of HP1α and its overexpression in breast cancer cells lines prompted the inventors to study HP1α expression in the physiological context of human cancer. First, data from published transcriptome studies performed in different tissue types, were analyzed using the Oncomine database (Rhodes et al, 2004). These data showed that HP1α is significantly and consistently overexpressed in several types of solid tumors, as well as in leukemia (Fig 9A) and that its expression is correlated with the expression of its binding partner CAF-1 p150 and the proliferation marker Ki67 (Fig 9B).

The present results in cultured cells systematically showed that the difference in HP 1α protein levels was more pronounced than the corresponding mRNA levels (Fig 1 and 4), possibly reflecting a post-translational regulation. This encouraged the inventors to analyze HP1α protein levels in frozen tumoral and non-tumoral human tissue sections by immunohistochemistry. An intense HP1α staining was systematically observed in tumoral cell nuclei in pancreas, uterus, ovary, prostate and breast malignancies (Fig 10A). In the corresponding non-tumoral tissues, HP1α levels were mostly below the limit of detection. This differential expression is specific for HP1α, since HP1β and γ show nuclear staining both in tumoral and non-tumoral tissues (Fig 10B). The nuclei showing intense HP1α imunostaining correspond to carcinoma cells, which also stain positive for the epithelial cytokeratin marker KL-1 (Fig 10C). In addition, a polyclonal antibody against HP1α, which provides a more intense staining than the monoclonal antibody used in figure 10, also showed a clear overexpression in carcinoma cells with some staining in non-tumoral tissue (Fig 11A). Staining for H3K9me3, however, did not show a clear difference between non-tumoral and tumoral tissues (Fig 11B), suggesting either that the HP1α detection is more sensitive or that the overexpression occurs independently from H3K9me3.

To determine the importance of HP1α overexpression for tumor growth and disease outcome, cryopreserved breast carcinoma specimens that were collected in 1995, were selected. The inventors focused on node negative and metastasis-free invasive carcinoma of which the size (median 18mm ; range 6-50mm) permitted primary conservative tumorectomy. These cases would benefit from new prognostic markers for the indication of adjuvant chemotherapy. Patient's and tumor characteristics are provided in Table I as presented below.

**Table I: Description of the patient samples:**

| **Age** | median: 53 (range: 26-70) | |
|---|---|---|
| **Menopaused** | yes | 54% |
| | no | 46% |
| **Histological type 9%** | Ductal | 88% |
| | Lobular | 9% |
| | Papillary | 1% |
| | Tubular | 1% |
| **Size classification** | T0/T1 | 62% |
| | T2 | 38% |

| **Tumor size (mm)** | median: 18 (range: 6-50) | |
|---|---|---|
| **Grade EE** | I | 33% |
| | II | 42% |
| | III | 25% |
| **Estrogen receptor** | positive | 86% |
| | negative | 14% |
| **Progesteron receptor** | positive | 69% |
| | negative | 31 % |

| **Mitotic index** | median: 8 (range: 0-105) | |
|---|---|---|
| **Ki67** | 15 - 40 | 52% |
| | <= 15 | 24% |
| | > 40 | 24% |
| **Adjuvant chemotherapy** | yes | 93% |
| | no | 7% |

The median follow-up is 146 months (range 30-161). At 10 years, overall survival, distant recurrence and disease progression rates were 90% [83-97%], 87% [80-95%] and 70% [61-81%], respectively. HP1α mRNA expression levels were measured by quantitative RT-PCR in 86 samples and expression levels were normalized to the reference gene ribosomal protein P0-like protein (RPLPO) (de Cremoux et al, 2004). The inventors found that high levels of HP1α tend to associate with increased age of the patient (p=0.0014) and larger, highly mitotic, grade III tumors (non-significant) (Fig 12A). In univariate analysis, HP1α continuous expression was significantly associated with an increased risk of disease progression: a one-unit increase of log2(HP1α) increased the risk of disease progression with 3% (RR = 1.03 [1.00-1.05], p=0.041). The inventors determined a cut-off value of 10, which divided patients in two groups (74.4% with HP1α levels ≤10 and 25.6% with HP1α levels >10) and was significantly associated with disease progression (p= 0.0113; Relative Risk= 2.47 [1.20-5.09]). At ten years, 75% [64-87] of the patients with low HP1α levels vs. 57% [39-83] of the patients with high HP1α levels had not shown disease progression. Moreover, this cut-off is also significantly associated with overall survival (p= 0.0134; Relative Risk= 3.76 [1.03-13.7]) and the occurrence of distant recurrences (p=0.011; Relative Risk= 3.74 [1.26-11.2]) (Fig 12B).

In multivariate analyses, adjusted for known prognostic factors (i.e. patient's age, mitotic index, tumor grade, tumor size, hormone receptor status and Ki67 levels), only HP1α expression is an independent prognostic factor for overall survival (p= 0.0431): high (>10) HP1α levels are associated with an increased risk of death (Relative Risk= 3.76 [1.03-13.7]). Similarly, adjusted for the same parameters, only high HP1α expression (p= 0.0077; Relative Risk= 3.02 [1.38-6.63]) and high tumor grade (p= 0.0170; Relative Risk for grade III= 4.53 [1.51-13.6]) are pejorative for disease progression.

In conclusion, the present results of immunohistochemistry demonstrate that HP1α, but not β or γ, is overexpressed in multiple types of human cancer cells. Furthermore, quantitative RT-PCR analysis carried out for HP1α showed significant correlation with clinopathological data and disease outcome. In the present series of 86 small breast tumors, high HP1α expression remained the only independent prognostic factor for overall survival after adjustment for classical prognostic markers, such as the mitotic index, tumor grade, tumor size, hormone receptor status or Ki67. Taken together, these data demonstrate that HP1α constitutes a new chromatin-related marker of cell proliferation and tumorigenesis of clinical relevance for prognosis of breast cancer and other types of cancer.

### DISCUSSION

### A unique regulation of the HP1α isoform

The HP1 family of proteins has been identified more than 20 years ago (James & Elgin, 1986), but the common or divergent functions of the three isoforms remain largely unknown. The present results show that in human cells, the HP1α isoform has unique properties, not shared by HP1β and γ. The protein is expressed in a proliferation-dependent manner, being downregulated during transient cell cycle exit. In line with these findings, cultured cancer cells overexpress HP1α compared to non-tumoral cells. Importantly, this is validated in patient samples. These data thus demonstrate a unique regulation of HP1α, which is not paralleled by HP1β or γ.

### HP1α importance in cancer

To evaluate the clinical importance of HP1α expression, HP1α protein and mRNA levels were analyzed in human cancer by immunohistochemistry and quantitative RT-PCR. Staining of cryopreserved human tissue samples showed a significant overexpression of HP1α, but not HP1β or γ, in tumoral cells. Thus, HP 1α constitutes a marker for diagnosis of multiple types of cancer. In contrast to other proliferation markers, such as Ki67, HP1α stains all tumoral cells and is thus highly suitable to determine the exact localization and extent of the tumor.

In order to quantify HP1α expression and determine its prognostic value, HP1α mRNA levels were measured in 86 small breast tumors with >10 years follow-up. The present data reveal that high HP1α expression correlates with decreased survival and increased occurrence of metastasis over time. Furthermore, multivariate analyses demonstrate that HP1α levels predict disease outcome better than standard prognostic markers. Thus, HP1α constitutes a marker of prognostic value, in breast cancer and in other types of cancer.

### REFERENCES

Andersson A, Ritz C, Lindgren D, Eden P, Lassen C, Heldrup J, Olofsson T, Rade J, Fontes M, Porwit-Macdonald A, Behrendtz M, Hoglund M, Johansson B, Fioretos T (2007) Microarray-based classification of a consecutive series of 121 childhood acute leukemias: prediction of leukemic and genetic subtype as well as of minimal residual disease status. Leukemia 21(6): 1198-1203. Epub 2007 Apr 1195.
Auth T, Kunkel E, Grummt F (2006) Interaction between HP1alpha and replication proteins in mammalian cells. Exp Cell Res 28: 28
Baxter J, Sauer S, Peters A, John R, Williams R, Caparros ML, Arney K, Otte A, Jenuwein T, Merkenschlager M, Fisher AG (2004) Histone hypomethylation is an indicator of epigenetic plasticity in quiescent lymphocytes. Embo J 23(22): 4462-4472
Berglund P, Landberg G (2006) Cycline overexpression reduces infiltrative growth in breast cancer: yet another link between proliferation control and tumor invasion. Cell Cycle 5(6): 606-609. Epub 2006 Mar 2015.
Carroll JS, Prall OW, Musgrove EA, Sutherland RL (2000) A pure estrogen antagonist inhibits cyclin E-Cdk2 activity in MCF-7 breast cancer cells and induces accumulation of p130-E2F4 complexes characteristic of quiescence. J Biol Chem 275(49): 38221-38229
Cheutin T, McNairn AJ, Jenuwein T, Gilbert DM, Singh PB, Misteli T (2003) Maintenance of stable heterochromatin domains by dynamic HP1 binding. Science 299(5607): 721-725.
de Cremoux P, Bieche I, Tran-Perennou C, Vignaud S, Boudou E, Asselain B, Lidereau R, Magdelenat H, Becette V, Sigal-Zafrani B, Spyratos F (2004) Inter-laboratory quality control for hormone-dependent gene expression in human breast tumors using real-time reverse transcription-polymerase chain reaction. Endocr Relat Cancer 11(3): 489-495.
Dialynas GK, Terjung S, Brown JP, Aucott RL, Baron-Luhr B, Singh PB, Georgatos SD (2007) Plasticity of HP1 proteins in mammalian cells. J Cell Sci 120(Pt 19): 3415-3424. Epub 2007 Sep 3412.
Elston & Ellis, Pathological prognostic factors in breast cancer. I. The value of histological grade in breast cancer: experience from a large study with long-term follow-up, Histopathology, 2002
Festenstein R, Pagakis SN, Hiragami K, Lyon D, Verreault A, Sekkali B, Kioussis D (2003) Modulation of heterochromatin protein 1 dynamics in primary Mammalian cells. Science 299(5607): 719-721.
Gilbert N, Boyle S, Sutherland H, de Las Heras J, Allan J, Jenuwein T, Bickmore WA (2003) Formation of facultative heterochromatin in the absence of HP1. Embo J 22(20): 5540-5550.
Hackett AJ, Smith HS, Springer EL, Owens RB, Nelson-Rees WA, Riggs JL, Gardner MB (1977) Two syngeneic cell lines from human breast tissue: the aneuploid mammary epithelial (Hs578T) and the diploid myoepithelial (Hs578Bst) cell lines. J Natl Cancer Inst 58(6): 1795-1806.
Istomina NE, Shushanov SS, Springhetti EM, Karpov VL, Krasheninnikov IA, Stevens K, Zaret KS, Singh PB, Grigoryev SA (2003) Insulation of the chicken beta-globin chromosomal domain from a chromatin-condensing protein, MENT. Mol Cell Biol 23(18): 6455-6468.
James TC, Elgin SC (1986) Identification of a nonhistone chromosomal protein associated with heterochromatin in Drosophila melanogaster and its gene. Mol Cell Biol 6(11): 3862-3872.
Kellum R, Alberts BM (1995) Heterochromatin protein 1 is required for correct chromosome segregation in Drosophila embryos. J Cell Sci 108(Pt 4): 1419-1431.
Kirschmann DA, Lininger RA, Gardner LM, Seftor EA, Odero VA, Ainsztein AM, Earnshaw WC, Wallrath LL, Hendrix MJ (2000) Down-regulation of HP1Hsalpha expression is associated with the metastatic phenotype in breast cancer. Cancer Res 60(13): 3359-3363
Kirschner-Schwabe R, Lottaz C, Todling J, Rhein P, Karawajew L, Eckert C, von Stackelberg A, Ungethum U, Kostka D, Kulozik AE, Ludwig WD, Henze G, Spang R, Hagemeier C, Seeger K (2006) Expression of late cell cycle genes and an increased proliferative capacity characterize very early relapse of childhood acute lymphoblastic leukemia. Clin Cancer Res 12(15): 4553-4561.
Martini E, Roche DM, Marheineke K, Verreault A, Almouzni G (1998) Recruitment of phosphorylated chromatin assembly factor 1 to chromatin after UV irradiation of human cells. J Cell Biol 143(3): 563-575.
Murzina N, Verreault A, Laue E, Stillman B (1999) Heterochromatin dynamics in mouse cells: interaction between chromatin assembly factor 1 and HP1 proteins. Mol Cell 4(4): 529-540
Nielsen SJ, Schneider R, Bauer UM, Bannister AJ, Morrison A, O'Carroll D, Firestein R, Cleary M, Jenuwein T, Herrera RE, Kouzarides T (2001b) Rb targets histone H3 methylation and HP1 to promoters. Nature 412(6846): 561-565
Norwood LE, Moss TJ, Margaryan NV, Cook SL, Wright L, Seftor EA, Hendrix MJ, Kirschmann DA, Wallrath LL (2006) A requirement for dimerization of HP1Hsalpha in suppression of breast cancer invasion. J Biol Chem 28: 28
Obuse C, Iwasaki O, Kiyomitsu T, Goshima G, Toyoda Y, Yanagida M (2004) A conserved Mis12 centromere complex is linked to heterochromatic HP1 and outer kinetochore protein Zwint-1. Nat Cell Biol 6(11): 1135-1141
Polo SE, Theocharis SE, Klijanienko J, Savignoni A, Asselain B, Vielh P, Almouzni G (2004) Chromatin assembly factors-1, a marker of clinical value to distinguish quiescent from proliferating cells. Cancer Res 64(7): 2371-2381
Pyeon D, Newton MA, Lambert PF, den Boon JA, Sengupta S, Marsit CJ, Woodworth CD, Connor JP, Haugen TH, Smith EM, Kelsey KT, Turek LP, Ahlquist P (2007) Fundamental differences in cell cycle deregulation in human papillomavirus-positive and human papillomavirus-negative head/neck and cervical cancers. Cancer Res 67(10): 4605-4619.
Quade BJ, Wang TY, Sornberger K, Dal Cin P, Mutter GL, Morton CC (2004) Molecular pathogenesis of uterine smooth muscle tumors from transcriptional profiling. Genes Chromosomes Cancer 40(2): 97-108.
Quivy JP, Gerard A, Cook AJ, Roche D, Almouzni G (2008) The HP1-p15O/CAF-1 interaction is required for pericentric heterochromatin replication and S-phase progression in mouse cells. Nat Struct Mol Biol
Quivy JP, Roche D, Kirschner D, Tagami H, Nakatani Y, Almouzni G (2004) A CAF-1 dependent pool of HP1 during heterochromatin duplication. Embo J 23(17): 3516-3526
Ramaswamy S, Ross KN, Lander ES, Golub TR (2003) A molecular signature of metastasis in primary solid tumors. Nat Genet 33(1): 49-54. Epub 2002 Dec 2009.
Regnier V, Vagnarelli P, Fukagawa T, Zerjal T, Bums E, Trouche D, Earnshaw W, Brown W (2005) CENP-A is required for accurate chromosome segregation and sustained kinetochore association of BubR1. Mol Cell Biol 25(10): 3967-3981.
Rhodes DR, Yu J, Shanker K, Deshpande N, Varambally R, Ghosh D, Barrette T, Pandey A, Chinnaiyan AM (2004) ONCOMINE: a cancer microarray database and integrated data-mining platform. Neoplasia 6(1): 1-6.
Richardson AL, Wang ZC, De Nicolo A, Lu X, Brown M, Miron A, Liao X, Iglehart JD, Livingston DM, Ganesan S (2006) X chromosomal abnormalities in basal-like human breast cancer. Cancer Cell 9(2): 121-132.
Ritou E, Bai M, Georgatos SD (2007) Variant-specific patterns and humoral regulation of HP1 proteins in human cells and tissues. J Cell Sci 120(Pt 19): 3425-3435. Epub 2007 Sep 3412.
Ryan RF, Schultz DC, Ayyanathan K, Singh PB, Friedman JR, Fredericks WJ, Rauscher FJ, 3rd (1999) KAP-1 corepressor protein interacts and colocalizes with heterochromatic and euchromatic HP1 proteins: a potential role for Kruppel-associated box-zinc finger proteins in heterochromatin-mediated gene silencing. Mol Cell Biol b(6): 4366-4378
Taddei A, Maison C, Roche D, Almouzni G (2001) Reversible disruption of pericentric heterochromatin and centromere function by inhibiting deacetylases. Nat Cell Biol 3(2): 114-120
Wang C, Ivanov A, Chen L, Fredericks WJ, Seto E, Rauscher FJ, 3rd, Chen J, Cress WD, Ryan RF, Schultz DC, Ayyanathan K, Singh PB, Friedman JR (2005) MDM2 interaction with nuclear corepressor KAP1 contributes to p53 inactivation. Embo J 24(18): 3279-3290. Epub 2005 Aug 3218.
Wang C, Rauscher FJ, 3rd, Cress WD, Chen J (2007) Regulation of E2F1 function by the nuclear corepressor KAP1. J Biol Chem 282(41): 29902-29909. Epub 22007 Aug 29917.
Williams L, Grafi G (2000) The retinoblastoma protein - a bridge to heterochromatin. Trends Plant Sci 5(6): 239-240.
Yu YP, Landsittel D, Jing L, Nelson J, Ren B, Liu L, McDonald C, Thomas R, Dhir R, Finkelstein S, Michalopoulos G, Becich M, Luo JH (2004) Gene expression alterations in rostate cancer predicting tumor aggression and preceding development of malignancy. J Clin Oncol 22(14): 2790-2799.

## Claims

1. An *in vitro* method for predicting or monitoring clinical outcome of a subject affected with a cancer, wherein the method comprises the step of determining the expression level of HP1α in a cancer sample from said subject, a high expression level of HP1α being indicative of a poor prognosis.

2. An *in vitro* method for diagnosing a cancer in a subject, wherein the method comprises the step of determining the expression level of HP1α in a sample from said subject, a high expression level of HP1α indicating that said subject suffers from a cancer.

3. An *in vitro* method for selecting a subject affected with a cancer for an adjuvant chemotherapy and/or radiotherapy or determining whether a subject affected with a cancer is susceptible to benefit from an adjuvant chemotherapy and/or radiotherapy, wherein the method comprises the step of determining the expression level of HP1α in a cancer sample from said subject, a high expression level of HP1α indicating that an adjuvant chemotherapy and/or radiotherapy is required.

4. An *in vitro* method for monitoring the response to a treatment of a subject affected with a cancer, wherein the method comprises the step of determining the expression level of HP1α in a cancer sample from said subject before the administration of the treatment, and in a cancer sample from said subject after the administration of the treatment, a decreased expression level of HP1α in the sample obtained after the administration of the treatment indicating that the subject is responsive to the treatment.

5. The method according to any one of claims 1 to 4, wherein the expression level of HP1α is determined by measuring the quantity of HP1α protein or HP1α mRNA.

6. The method according to claim 5, wherein the quantity of HP1α protein is measured by immunohistochemistry or semi-quantitative Western-Blot.

7. The method according to claim 5, wherein the quantity of HP1α mRNA is measured by quantitative or semi-quantitative RT-PCR, or by real time quantitative or semi-quantitative RT-PCR

8. The method according to any one of claims 1 to 7, wherein the method further comprises the step of comparing the expression level of HP1α to a reference expression level.

9. The method according to claim 8, wherein the reference expression level is the expression level of HP1α in a normal sample.

10. The method according to claim 1 and 3 to 7, wherein the method further comprises the step of comparing the expression level of HP1α to a reference expression level and wherein the reference expression level is the expression level of a gene having a stable expression in different cancer samples, such as RPLPO gene.

11. The method according to any one of claims 8 to 10, wherein the method further comprises the step of determining whether the expression level of HP1α is high compared to said reference expression level.

12. The method according to any one of claims 1 and 5 to 11, wherein a poor prognosis is a decreased patient survival and/or an early disease progression and/or an increased disease recurrence and/or an increased metastasis formation.

13. The method according to any one of claims 1 to 11, further comprising assessing at least one another cancer or prognosis markers such as tumor grade, hormone receptor status, mitotic index, tumor size or expression of proliferation markers such as Ki67, MCM2, CAF-1 p60 and CAF-1 p150.

14. The method according to any one of claims 1 to 13, wherein the cancer is a solid cancer or a hematopoietic cancer, preferably a solid cancer and more preferably an early stage solid cancer without local or systemic invasion.

15. The method according to any one of claims 1 to 14, wherein the cancer is selected from the group consisting of breast cancer, pancreas cancer, uterus and cervix cancers, ovary cancer, prostate cancer and leukemia.

16. The method according to claim 15, wherein the cancer is breast cancer, preferably an early stage breast cancer without local or systemic invasion.
